(19) Europäisches Patentamt
European Patent Office
Office européen des brevets

(11) **EP 4 442 735 A1**

(12) **EUROPEAN PATENT APPLICATION**
published in accordance with Art. 153(4) EPC

(43) Date of publication:
**09.10.2024 Bulletin 2024/41**

(21) Application number: **22900913.9**

(22) Date of filing: **11.10.2022**

(51) International Patent Classification (IPC):
**C08J 3/12** (2006.01)     **A61F 13/53** (2006.01)
**B01J 20/26** (2006.01)     **B01J 20/28** (2006.01)

(52) Cooperative Patent Classification (CPC):
**A61F 13/53; B01J 20/26; B01J 20/28; C08J 3/12**

(86) International application number:
**PCT/JP2022/037928**

(87) International publication number:
**WO 2023/100479 (08.06.2023 Gazette 2023/23)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **30.11.2021 JP 2021193913**

(71) Applicant: **Sumitomo Seika Chemicals Co., Ltd. Kako-gun, Hyogo 675-0145 (JP)**

(72) Inventors:
• **SANO, Kentaro**
  **Himeji-shi, Hyogo 672-8076 (JP)**
• **SAWAKI, Hiroki**
  **Himeji-shi, Hyogo 672-8076 (JP)**

(74) Representative: **Vossius & Partner Patentanwälte Rechtsanwälte mbB Siebertstrasse 3 81675 München (DE)**

(54) **WATER ABSORBENT RESIN PARTICLES AND ABSORBENT**

(57)     One aspect of the present invention relates to water absorbent resin particles in which an elasticity is 60 to 200 [kPa/mm] when compressed at 24 kPa to 96 kPa after immersion in physiological saline for 2 minutes, and a water retention capacity of physiological saline is 30 to 60 [g/g].

*Fig.1*

EP 4 442 735 A1

## Description

## Technical Field

The present invention relates to water absorbent resin particles and an absorbent.

## Background Art

In recent years, water absorbent resin particles have been widely used in various fields such as sanitary materials such as disposable diapers and sanitary products, agricultural and horticultural materials such as water retention agents and soil conditioners, and industrial materials such as water sealants and anti-condensation agents. Among these fields, in particular, use in sanitary materials such as disposable diapers, sanitary products, and portable toilets is common. In relation to water absorbent resin particles, Patent Literature 1 discloses water-containing water absorbent polymer particles having a predetermined strength. Patent Literature 2 discloses a super water absorbent resin in which the use amounts of a substance to be crosslinked and a crosslinking agent are adjusted in a predetermined range. Patent Literature 3 discloses a method for obtaining a super water absorbent resin by a method in which a certain amount of inorganic powder, a certain amount of an ethylene-acrylic acid copolymer, and a certain amount of water are mixed with a super water absorbent resin, and then this mixture is dried.

## Citation List

## Patent Literature

[Patent Literature 1] Japanese Unexamined Patent Publication No. 2013-95822
[Patent Literature 2] Japanese Unexamined Patent Publication No. H8-53550
[Patent Literature 3] Japanese Unexamined Patent Publication No. H8-113653

## Summary of Invention

## Technical Problem

In absorbent articles such as disposable diapers and sanitary products, there is a demand for absorbents having a liquid diffusing capacity in order to use the products effectively. Absorbents that do not have a sufficient diffusing capacity tend to be discarded in a state in which a liquid pool is formed in the center of the absorbent despite a large number of water absorbent resin particles that have not expressed a water-absorbing function remain in the absorbent. Therefore, when it is possible to carry out the diffusion over a wider range, more of the water absorbent resin particles will function and it will be possible to use the entire absorbent without waste.

One factor that inhibits diffusion in the absorbent is blocking by swollen water absorbent resin particles. Generally, in order to reduce this blocking, the strength in a state of swelling is improved by strengthening the crosslinking. However, when the crosslinking is strengthened, the capacity of the absorbent is decreased at the same time and thus the re-wet amount after liquid absorption may increase.

The present invention has an object of providing water absorbent resin particles that provide an absorbent that has better liquid diffusivity after liquid absorption and is able to suppress re-wet after liquid absorption.

## Solution to Problem

According to the findings of the present inventors, re-wet after liquid absorption in an absorbent article is comparatively likely to occur when used by 1- to 2-year-old children. The present inventors assume that one reason for this is that 1- to 2-year-old children have not yet fully developed the ability to walk on their own and infants frequently fall on their buttocks, thus, the water absorbent resin particles included in the absorbent are easily strongly compressed. On the basis of this assumption, the present inventors verified the pressure applied to water absorbent resin particles when an active 1- to 2-year-old child uses an absorbent article, discovered that this pressure was 24 kPa to 96 kPa, and found that, by setting the gel strength in a state where pressure is applied in this range to a specific range and setting the water retention capacity of physiological saline of the water absorbent resin particles to a specific range, it is possible to obtain water absorbent resin particles that provide an absorbent that has better diffusivity and is able to suppress re-wet after liquid absorption.

One aspect of the present invention relates to water absorbent resin particles, having an elasticity of 60 to 200 [kPa/mm] when compressed at 24 kPa to 96 kPa after immersion in physiological saline for 2 minutes, and a water retention capacity of physiological saline of 30 to 60 [g/g].

Another aspect of the present invention relates to an absorbent including the above-mentioned water absorbent resin particles.

**Advantageous Effects of Invention**

According to the present invention, it is possible to provide water absorbent resin particles that provide an absorbent that has better liquid diffusivity after liquid absorption and is able to suppress re-wet after liquid absorption.

**Brief Description of Drawings**

Fig. 1 is a graph for illustrating an elasticity calculation method.
Fig. 2 is a schematic cross-sectional view showing one embodiment of an absorbent article.
Fig. 3 is a diagram for illustrating a diffusion distance evaluation method.
Fig. 4 is a diagram showing a measurement device for measuring a water absorption amount under load.

**Description of Embodiments**

A detailed description will be given below of several embodiments of the present invention. However, the present invention is not limited to the following embodiments.

In the present specification, "acrylic" and "methacrylic" are collectively denoted as "(meth)acrylic". "Acrylate" and "methacrylate" are also similarly denoted as "(meth)acrylate". In the numerical ranges described stepwise in the present specification, it is possible to arbitrarily combine the upper limit value or lower limit value of the numerical range in a particular step with the upper limit value or lower limit value of the numerical range in another step. In the numerical ranges described in the present specification, the upper limit value or lower limit value of the numerical ranges may be replaced with the values shown in the Examples. The materials exemplified in the present specification may be used alone or in a combination of two or more. In a case where there are a plurality of substances corresponding to each component present in the composition, the content of each component in the composition means the total amount of the plurality of substances present in the composition unless otherwise specified. In the present specification, "physiological saline" is a sodium chloride aqueous solution having a concentration of 0.9% by mass and the concentration of 0.9% by mass is a concentration based on the mass of the physiological saline.

The water absorbent resin particles according to the present embodiment have an elasticity of 60 to 200 [kPa/mm] when compressed at 24 kPa to 96 kPa after immersion in physiological saline for 2 minutes. To have a better effect of improving the diffusivity of the liquid after liquid absorption and effect of suppressing re-wet after liquid absorption, the lower limit of the elasticity when compressed at 24 kPa to 96 kPa after immersion in physiological saline for 2 minutes may be 70 kPa/mm or more, 80 kPa/mm or more, 90 kPa/mm or more, 100 kPa/mm or more, 110 kPa/mm or more, 120 kPa/mm or more, 130 kPa/mm or more, 140 kPa/mm or more, 150 kPa/mm or more, 160 kPa/mm or more, 170 kPa/mm or more, 180 kPa/mm or more, or 185 kPa/mm or more. To have a better effect of improving the diffusivity of the liquid after liquid absorption and effect of suppressing re-wet after liquid absorption, the upper limit of the elasticity when compressed at 24 kPa to 96 kPa after immersion in physiological saline for 2 minutes may be 190 kPa/mm or less, 180 kPa/mm or less, 170 kPa/mm or less, 160 kPa/mm or less, 150 kPa/mm or less, 140 kPa/mm or less, 130 kPa/mm or less, 120 kPa/mm or less, 110 kPa/mm or less, 100 kPa/mm or less, or 95 kPa/mm or less. The elasticity when compressed at 24 kPa to 96 kPa after immersion in physiological saline for 2 minutes is preferably 80 kPa/mm to 190 kPa/mm, 90 kPa/mm to 190 kPa/mm, 140 kPa/mm to 180 kPa/mm, or 150 kPa/mm to 180 kPa/mm.

The elasticity when compressed at 24 kPa to 96 kPa after immersion in physiological saline for 2 minutes is measured using the following steps (1) to (5).

(1) 0.200 g of water absorbent resin particles are precisely weighed into an acrylic cylinder having an inner diameter of 2.0 cm and a depth of 5.0 cm, in which the bottom surface is lined with nylon mesh (Nippon Tokushu fabric Inc., NNo. 250T), the water absorbent resin particles are spread evenly over the entire bottom surface, and 13 g of physiological saline is poured in from the top of the acrylic cylinder in a state where no liquid flows out from the bottom of the cylinder (nylon mesh side).
(2) Two minutes after pouring in the physiological saline, the acrylic cylinder is lifted up and the excess physiological saline that was not absorbed is discharged from the bottom of the cylinder to obtain particles where liquid absorption occurred (sample for elasticity evaluation). Two minutes after pouring in the physiological saline corresponds to the approximate time that the water absorbent resin particles are immersed in urine in the absorbent during three urinations.

(3) An acrylic column having a diameter of 2 cm and a height of 6 cm is connected to the load cell of a small tabletop tester (SHIMADZU, EZ-TEST).

(4) The sample for elasticity evaluation is compressed inside the acrylic cylinder from above in the vertical direction at a speed of 1 cm/min using the load cell. The compression of the sample for elasticity evaluation is continued until the stress from the sample for elasticity evaluation corresponds to 45N.

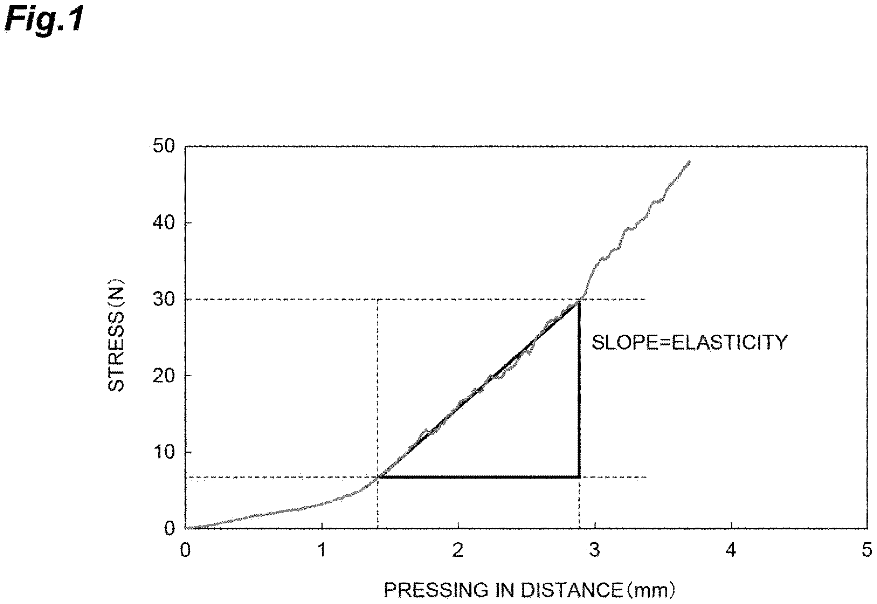

(5) The distance and stress when pressing in the sample for elasticity evaluation are plotted. Fig. 1 is a graph for illustrating an elasticity calculation method. In Fig. 1, the horizontal axis is the distance (unit: mm) that the sample for elasticity evaluation was pressed in and the vertical axis is the stress (unit: N). As shown in Fig. 1, the elasticity (kPa/mm) is calculated from the slope of a straight line connecting the pressing in distance at a stress of 24 kPa (7.5 N) and the pressing in distance at a stress of 96 kPa (30 N). The stress of 24 kPa corresponds to the local pressure between the buttocks and the ground surface when a 2-year-old child of average weight sits thereon and the stress of 96 kPa corresponds to an impact that may occur when the child falls on his or her buttocks.

It is possible for the elasticity when compressed at 24 kPa to 96 kPa after immersion in physiological saline for 2 minutes to be adjusted in the above-mentioned range through, for example, the type and use amount of the material for manufacturing the water absorbent resin particles, or the like. As a method for obtaining water absorbent resin particles for which the elasticity is in the above-mentioned range when compressed at 24 kPa to 96 kPa after immersion in physiological saline for 2 minutes, examples include a method in which a coating layer is provided on at least one part of the surface of the water absorbent resin particles using a coating material. It is possible for the elasticity when compressed at 24 kPa to 96 kPa after immersion in physiological saline for 2 minutes to be adjusted in the above-mentioned range by adjusting the degree of hydrophilicity of the coating layer by esterification modification or the like (generally, the elasticity tends to increase when the coating layer is hydrophobic), adjusting the amount of the coating layer (coating material use amount) on the water absorbent resin (generally, the elasticity tends to increase when the coating material use amount is large), providing a coating layer using a coating material having a high-breaking strength (generally, the elasticity tends to increase when a coating material having a high-breaking strength is used), a method combining any of the above, or the like. Regardless of the presence or absence of a coating layer, in a case where the water absorbent resin particles include the polymer particles described below, it is also possible to adjust the elasticity in the above-mentioned range by adjusting the neutralization degree (generally, the elasticity tends to increase when the neutralization degree is low), adjusting the degree of hydrophilicity of the surface of the water absorbent resin particles by esterification modification or the like (generally, the elasticity tends to increase when the water absorbent resin particles are hydrophobic), or a combination of any of the above.

The water absorbent resin particles according to the present embodiment have a water retention capacity of physiological saline of 30 to 60 [g/g]. To have a better effect of improving the diffusivity of the liquid after liquid absorption and effect of suppressing re-wet after liquid absorption, the lower limit of the water retention capacity of physiological saline may be 31 g/g or more, 32 g/g or more, 33 g/g or more, 34 g/g or more, 35 g/g or more, 36 g/g or more, 37 g/g or more, 38 g/g or more, or 39 g/g or more. To have a better effect of improving the diffusivity of the liquid after liquid absorption and effect of suppressing re-wet after liquid absorption, the lower limit of the water retention capacity of physiological saline may be 58 g/g or less, 56 g/g or less, 54 g/g or less, 52 g/g or less, 50 g/g or less, 48 g/g or less, 46 g/g or less, 44 g/g or less, 42 g/g or less, or 40 g/g or less. The water retention capacity of physiological saline is measured by the method described in the Examples below. The water retention capacity of physiological saline is preferably 35 g/g to 60 g/g or 36 g/g to 54 g/g.

The water absorption amount of the water absorbent resin particles according to the present embodiment with respect to physiological saline under a load of 0.9 psi may be, for example, 8.0 g/g to 18.0 g/g, 9.0 g/g to 18.0 g/g, 9.0 g/g to 17.0 g/g, 10.0 g/g to 17.0 g/g, or 10.0 g/g to 16.0 g/g. The water absorption amount with respect to the physiological saline under a load of 0.9 psi is measured by the method described in the Examples below.

The median particle diameter of the water absorbent resin particles according to the present embodiment may be, for example, 100 μm to 800 μm, 150 μm to 700 μm, 200 μm to 600 μm, 250 μm to 500 μm, 100 μm to 400 μm, 100 μm to 360 μm, 200 μm to 400 μm, 200 μm to 360 μm, 250 μm to 400 μm, or 250 μm to 360 μm. The median particle diameter of the water absorbent resin particles is measured by the method described in Examples below.

The shape of the water absorbent resin particles is not particularly limited and may be, for example, approximately spherical, crushed, or granular, or may be formed by the aggregation of primary particles having these shapes.

The water absorbent resin particles may include polymer particles. The polymer particles may be crosslinked polymers formed by polymerization of monomers including ethylenically unsaturated monomers. The polymer particles may include monomer units derived from ethylenically unsaturated monomers. It is possible to produce polymer particles, for example, using a method including a step for polymerizing a monomer including an ethylenically unsaturated monomer.

Examples of the polymerization method include a reverse phase suspension polymerization method, an aqueous solution polymerization method, a bulk polymerization method, a precipitation polymerization method, and the like.

The ethylenically unsaturated monomer may be a water-soluble ethylenically unsaturated monomer. The solubility of the water-soluble ethylenically unsaturated monomer in 100 g of water may be 1.0 g or more at 25°C. Examples of water-soluble ethylenically unsaturated monomers include (meth)acrylic acid and salts thereof, 2-(meth)acrylamide-2-methylpropanesulfonic acid and salts thereof, (meth)acrylamide, N,N-dimethyl(meth)acrylamide, 2-hydroxyethyl(meth)acrylate, N-methylol(meth)acrylamide, polyethylene glycol mono(meth)acrylate, N,N-diethylaminoethyl(meth)acrylate, N,N-diethylaminopropyl(meth)acrylate, and diethylaminopropyl(meth)acrylamide. In a case where the ethylenically unsaturated monomer has an amino group, the amino group may be quaternized. Ethylenically unsaturated monomers may be used alone or in a combination of two or more.

In a case where the ethylenically unsaturated monomer has an acid group, the acid group may be neutralized using an alkaline neutralizing agent before being used in the polymerization reaction. The neutralization degree in the ethylenically unsaturated monomer due to the alkaline neutralizing agent is, for example, 10 mol% to 100 mol% of the acid groups in the ethylenically unsaturated monomer, 50 mol% to 90 mol%, or 60 mol% to 80 mol%.

From the viewpoint of the ease of industrial availability, the ethylenically unsaturated monomer may include at least one compound selected from the group consisting of (meth)acrylic acid and salts thereof, acrylamide, methacrylamide, and N,N-dimethylacrylamide. The ethylenically unsaturated monomer may include at least one compound selected from the group consisting of (meth)acrylic acid and salts thereof, and acrylamide.

As the monomer for obtaining the water absorbent resin particles, a monomer other than the above-mentioned ethylenically unsaturated monomers may be used. It is possible to use such monomers, for example, in a mixture with an aqueous solution including the above-mentioned ethylenically unsaturated monomers. The use amount of ethylenically unsaturated monomer may be 60 mol% to 100 mol%, 70 mol% to 100 mol%, 80 mol% to 100 mol%, 90 mol% to 100 mol%, or 95 mol% to 100 mol%, with respect to the total amount of monomers. The ratio of (meth)acrylic acid and salts thereof may be 60 mol% to 100 mol%, 70 mol% to 100 mol%, 80 mol% to 100 mol%, 90 mol% to 100 mol%, or 95 mol% to 100 mol%, with respect to the total amount of monomers.

Although crosslinking occurs due to self-crosslinking during polymerization, crosslinking may be promoted by using an internal crosslinking agent. Using an internal crosslinking agent makes it easy to control the water-absorbent characteristics (water retention capacity and the like) of the water absorbent resin particles. The internal crosslinking agent is usually added to the reaction solution during the polymerization reaction.

The polymer in at least the surface layer portion of the polymer particles may be crosslinked by reaction with a surface crosslinking agent. The surface crosslinking agent may be, for example, a compound having two or more functional groups (reactive functional groups) that are reactive with functional groups derived from ethylenically unsaturated monomers.

In addition to the polymer of the ethylenically unsaturated monomer, the polymer particles may include a certain amount of water and may further include various additional components therein. Examples of additional components include gel stabilizers, metal chelating agents, and inorganic particles.

The particle size distribution of the water absorbent resin particles may be adjusted by performing operations such as particle size adjustment using classification using a sieve, as necessary. For example, a fraction that passes through a sieve having openings of 850 μm but does not pass through a sieve having openings of 250 μm may be used as the water absorbent resin particles.

The water absorbent resin particles may be coated resin particles having polymer particles and a coating layer coating at least a part of the surface of the polymer particles. It is possible to form the coating layer, for example, by a method including a step of coating at least a part of the polymer particles with a coating material to form a coating layer on at least a part of the surface of the polymer particles.

The coating layer may be formed of a polymer (homopolymer or copolymer). The coating layer may contain a polymer having a carboxyl group. In a polymer having a carboxyl group, the carboxyl group may be neutralized using an alkaline neutralizing agent, or may not be neutralized. The polymer having a carboxyl group includes a monomer unit derived from a monomer having a carboxyl group. Examples of the monomer having a carboxyl group include (meth)acrylic acid and salts thereof, and the like. Examples of (meth)acrylic acid salts include sodium acrylate, potassium acrylate, and the like.

The polymer included in the coating layer may include a monomer unit derived from a monomer (other monomer) other than the monomer having a carboxyl. The other monomers may be, for example, substituted or unsubstituted alkenes.

Examples of unsubstituted alkenes include ethylene, propylene, and butene. Examples of substituted alkenes include styrene.

The polymer included in the coating layer may be a polymer only including monomer units derived from monomers having a carboxyl group and may be at least one type selected from the group consisting of poly(meth)acrylic acid and salts thereof.

The polymer included in the coating layer may be a copolymer including monomer units derived from a monomer having a carboxyl group and a substituted or unsubstituted alkene. The copolymer may be, for example, a copolymer including monomer units derived from (meth)acrylic acid or a salt thereof and ethylene and may be a copolymer including monomer units derived from (meth)acrylic acid or a salt thereof and styrene.

The ratio of the coating layer in the coated resin particles may be, for example, 1 part by mass to 40 parts by mass, with respect to 100 parts by mass of the polymer particles. The ratio of the coating layer in the coated resin particles may be, for example, 1 part by mass or more, 5 parts by mass or more, or 8 parts by mass or more and may be 40 parts by mass or less, 35 parts by mass or less, 30 parts by mass or less, or 25 parts by mass, with respect to 100 parts by mass of the polymer particles.

It is possible to obtain the coated resin particles by a method that includes coating at least a part of the polymer particles with a coating material to form a coating layer on at least a part of the surface of the polymer particles. The coating material may include a liquid medium and the like in addition to the constituent components of the above-mentioned coating layer. Examples of the liquid medium include water and ethers such as tetrahydrofuran.

The coating material may be, for example, a liquid including the above-mentioned polymer and a liquid medium in which the polymer is dissolved or dispersed.

It is possible to form the coating layer, for example, by (1) a method using an eggplant-shaped flask, (2) a method using a sprayer, or (3) a method using various granulators.

(1) Method Using Eggplant-Shaped Flask

In the method using an eggplant-shaped flask, first, the coating material is injected into the eggplant-shaped flask and then the water absorbent resin particles are injected therein. The eggplant-shaped flask is attached to an evaporator, heated while rotating, and the liquid medium included in the coating material is distilled off under reduced pressure conditions. Thus, coated resin particles are obtained in which the surfaces of the water absorbent resin particles are coated with the coating material.

(2) Method Using Sprayer

In the method using a sprayer, first, water absorbent resin particles are added to a separable flask equipped with a stirrer blade and stirred. A coating material is sprayed onto the water absorbent resin particles stirred up by the stirrer blade. It is possible to spray the coating material using, for example, a two-fluid type nozzle. In anticipation of an even coating, it is desirable for the coating material to be sprayed in mist form using an air stream of an inert gas such as nitrogen. Thereafter, the contents of the separable flask are taken out, heated in a hot air dryer, and then cooled to room temperature to obtain coated resin particles.

(3) Method Using Various Granulators

Examples of granulators used for producing coated resin particles include a rolling granulator, a stirring granulator, and a fluidized bed granulator.

In a case of using a rolling granulator, an inclined shallow circular container equipped with the rolling granulator is rotated, the water absorbent resin particles are supplied to the circular container, and an appropriate amount of the coating material is added thereto. Then, due to the solvent or dispersion medium included in the coating material, some of the water absorbent resin particles being rolled are aggregated and a coating layer is formed on the surface thereof. It is possible to perform the step of adding the water absorbent resin particles and coating material a plurality of times as necessary.

In a case of using a stirring granulator, the water absorbent resin particles are injected into a mixer equipped in the stirring granulator, mixed by stirring, and the coating material is added thereto. Then, due to the liquid medium included in the coating material, some of the water absorbent resin particles being stirred are aggregated and a coating layer is formed on the surface thereof. It is possible to perform the step of adding the water absorbent resin particles and coating material a plurality of times as necessary. It is possible to suppress excessive aggregation of the water absorbent resin particles by controlling the shear force of the mixer.

In a case of using a fluidized bed granulator, first, water absorbent resin particles are injected into a container equipped with a fluidized bed granulator that is able to blow hot air from the bottom thereof and the water absorbent resin particles are fluidized preliminarily. Thereafter, when the coating material is sprayed from a nozzle provided in the container, due to the liquid medium included in the coating material, some of the water absorbent resin particles being stirred are aggregated and a coating layer is formed on the surface thereof. It is possible to perform the spraying of the coating material a plurality of times as necessary. It is possible to suppress excessive aggregation of the water absorbent resin particles by adjusting the spraying amount and spraying frequency of the coating material. As the fluidized bed granulator, for example, it is possible to use a fluidized bed granulator FBD/SG (manufactured by Yenchen Machinery).

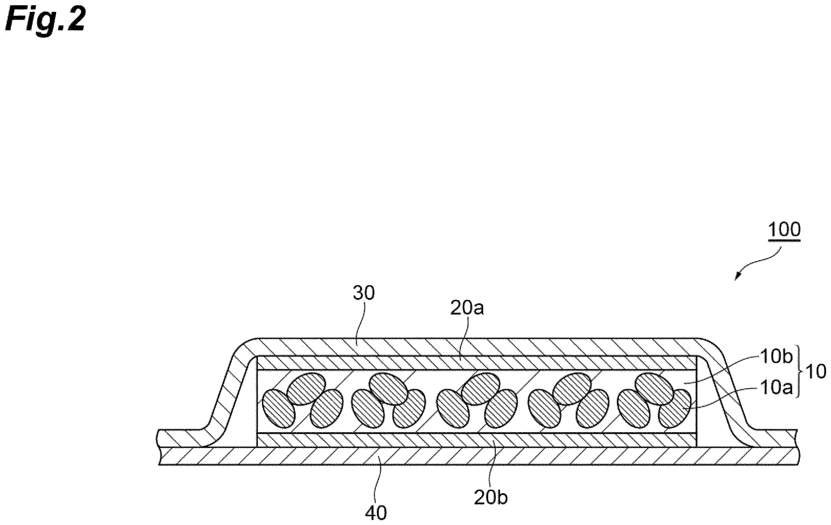

Water absorbent resin particles are used, for example, to form an absorbent that forms an absorbent article such as a diaper. Fig. 2 is a cross-sectional view showing an example of an absorbent article. An absorbent article 100 shown in Fig. 2 is provided with a sheet-shaped absorbent 10, core wraps 20a and 20b, a liquid permeable sheet 30, and a liquid impermeable sheet 40. In the absorbent article 100, the liquid impermeable sheet 40, the core wrap 20b, the absorbent 10, the core wrap 20a, and the liquid permeable sheet 30 are laminated in this order. Fig. 2 illustrates portions where there are gaps between the members, but the members may be in close contact with each other without such gaps being present.

The absorbent 10 has water absorbent resin particles 10a according to the above-mentioned embodiment and a fiber layer 10b including a fibrous material. The water absorbent resin particles 10a are dispersed in the fiber layer 10b. The water absorbent resin particles 10a may include, for example, the above-mentioned coated resin particles and other water absorbent resin particles. In this case, the content of the coated resin particles may be, for example, 5 parts by mass or more or 15 parts by mass or more and may be 95 parts by mass or less or 85 parts by mass or less, with respect to a total of 100 parts by mass of the coated resin particles and other water absorbent resin particles. The coated resin particles may be used in a mixture of a plurality of types of coated resin particles that differ in at least one condition selected from the group consisting of the thickness of the coating layer, the material of the coating layer, and the material of the polymer particles.

The core wrap 20a is arranged on one surface side of the absorbent 10 (on the upper side of the absorbent 10 in Fig. 2) in a state of contact with the absorbent 10. The core wrap 20b is arranged on the other surface side of the absorbent 10 (the lower side of the absorbent 10 in Fig. 2) in a state of contact with the absorbent 10. The absorbent 10 is arranged between the core wrap 20a and the core wrap 20b. Examples of the core wraps 20a and 20b include tissue, nonwoven fabric, and the like. The core wrap 20a and the core wrap 20b have, for example, main surfaces of the same size as the absorbent 10.

The liquid permeable sheet 30 is arranged at the outermost part on the side where the liquid to be absorbed enters. The liquid permeable sheet 30 is arranged on the core wrap 20a in a state of contact with the core wrap 20a. Examples of the liquid permeable sheet 30 include nonwoven fabrics, porous sheets, and the like formed of synthetic resins such as polyethylene, polypropylene, polyester, and polyamide. The liquid impermeable sheet 40 is arranged at the outermost part of the absorbent article 100 on the opposite side from the liquid permeable sheet 30. The liquid impermeable sheet 40 is arranged on the lower side of the core wrap 20b in a state of contact with the core wrap 20b. Examples of the liquid impermeable sheet 40 include sheets formed of synthetic resins such as polyethylene, polypropylene, and polyvinyl chloride, sheets formed of composite materials of these synthetic resins and nonwoven fabrics, and the like. The liquid permeable sheet 30 and the liquid impermeable sheet 40 have, for example, main surfaces wider than the main surface of the absorbent 10, and the outer edges of the liquid permeable sheet 30 and the liquid impermeable sheet 40 extend on the periphery of the absorbent 10 and core wraps 20a and 20b.

The magnitude relationships among the absorbent 10, the core wraps 20a and 20b, the liquid permeable sheet 30, and the liquid impermeable sheet 40 are not particularly limited and may be adjusted, as appropriate, depending on the use of the absorbent article or the like. The method for retaining the shape of the absorbent 10 using the core wraps 20a and 20b is not particularly limited and the absorbent may be wrapped with a plurality of core wraps as shown in Fig. 2 or the absorbent may be wrapped with a single core wrap.

According to one aspect of the present embodiment, there is provided a method for improving the diffusivity of a liquid after liquid absorption and suppressing re-wet after liquid absorption in an absorbent including water absorbent resin particles, the method including adjusting the elasticity of the water absorbent resin particles to 60 to 200 [kPa/mm] when compressed at 24 kPa to 96 kPa after immersion in physiological saline for 2 minutes, and adjusting the water retention capacity of physiological saline of the water absorbent resin particles to 30 to 60 [g/g]. For specific embodiments of the method, it is possible to apply the above-mentioned embodiments.

**Examples**

A more detailed description will be given below of the present invention with reference to Examples. However, the present invention is not limited to these Examples.

Comparative Example 1

A round-bottomed cylindrical separable flask having an inner diameter of 11 cm and a volume of 2 L and equipped with a reflux cooling device, a dropping funnel, a nitrogen gas introduction tube, and a stirrer (having stirrer blades in which four inclined paddle blades having a blade diameter of 5 cm are set in two stages) was prepared. 293 g of n-heptane (hydrocarbon dispersion medium) and 0.736 g of a maleic anhydride-modified ethylene-propylene copolymer (polymeric dispersant, Mitsui Chemicals, Inc., Hi-Wax 1105A) were added to this separable flask to obtain a mixture. The dispersant was dissolved by heating the mixture to 80°C while stirring at a rotation speed of 300 rpm and then the mixture was cooled to 55°C.

Subsequently, 92.0 g of an 80.5% by mass acrylic acid aqueous solution (acrylic acid: 1.03 mol) was put into a triangular flask having a volume of 500 mL. Subsequently, while cooling from the outside, 75 mol% of the acrylic acid was neutralized by the dropwise addition of 102.2 g of a 30% by mass sodium hydroxide aqueous solution. Subsequently, 0.092 g of hydroxylethyl cellulose (thickener, SUMITOMO SEIKA CHEMICALS CO., LTD., HEC AW-15F), 0.0736 g (0.272 mmol) of potassium persulfate (water-soluble radical polymerization initiator), 0.0101 g (0.0581 mmol) of ethylene glycol diglycidyl ether (internal crosslinking agent), and 32.85 g of ion-exchanged water were added and then dissolved to prepare a first stage monomer aqueous solution.

After adding the above-mentioned first stage monomer aqueous solution to the above-mentioned separable flask, stirring was carried out for 10 minutes. Thereafter, 7.356 g of a surfactant solution obtained by heat-dissolving 0.736 g of sucrose stearate (surfactant, manufactured by Mitsubishi-Chemical Foods Corporation, Ryoto Sugar Ester S-370, HLB: 3) in 6.62 g of n-heptane was added to the separable flask to obtain a reaction solution. Then, while stirring the reaction solution at a rotation speed of 550 rpm, the inside of the system was sufficiently substituted with nitrogen. Thereafter, the separable flask was immersed in a water bath at 70°C to heat the reaction solution and first stage polymerization was performed for 10 minutes to obtain a first stage reaction mixture.

Subsequently, 128.8 g (acrylic acid: 1.44 mol) of an 80.5% by mass acrylic acid aqueous solution was put into another triangular flask having a volume of 500 mL. Subsequently, while cooling from the outside, 75 mol% of acrylic acid was neutralized by the dropwise addition of 143.1 g of a 30% by mass sodium hydroxide aqueous solution. Thereafter, 0.1030 g (0.3812 mmol) of potassium persulfate, 0.0116 g (0.0655 mmol) of ethylene glycol diglycidyl ether (internal crosslinking agent), and 0.63 g of ion-exchanged water were added and then dissolved to prepare a second stage monomer aqueous solution.

Then, after cooling the above-mentioned first stage reaction mixture to 25°C while stirring at a rotation speed of 1000 rpm, the total amount of the above-mentioned second stage monomer aqueous solution was added to the first stage reaction mixture to obtain a reaction solution. Then, while stirring the reaction solution, the inside of the system was sufficiently substituted with nitrogen. Thereafter, the separable flask was immersed in a water bath at 70°C to heat the reaction solution and second stage polymerization was performed for 5 minutes to obtain a second stage reaction mixture (polymer particles before surface crosslinking).

After the second stage polymerization, the second stage reaction mixture was heated in an oil bath at 125°C and 267 g of water was extracted to the outside of the system by azeotropic distillation of n-heptane and water while refluxing the n-heptane. Subsequently, 0.0884 g (0.5075 mmol) of ethylene glycol diglycidyl ether was added as a surface crosslinking agent and then the result was held at 83°C for 2 hours to obtain a dispersion liquid of polymer particles after surface crosslinking.

Thereafter, the dispersion liquid of resin particles after the above-mentioned surface crosslinking was heated in an oil bath at 125°C and dried by evaporating the n-heptane to obtain a dried product. The dried product was passed through a sieve having openings of 850 μm to obtain 232.8 g of polymer particles A in the form of aggregated spherical particles.

The above operation was repeated and the collected water absorbent resin particles were classified using a sieve with openings of 250 μm to obtain 500 g or more of water absorbent resin particles A having a particle size of 250 μm to 850 μm (median particle diameter of 341 μm).

Comparative Example 2

500 g or more of water absorbent resin particles B (median particle diameter 335 μm) were obtained in the same manner as in Comparative Example 1 except that 0.092 g (0.339 mmol) of 2,2'-azobis(2-amidinopropane) dihydrochloride was added as the azo-based compound in the first stage monomer aqueous solution, the addition amount of potassium persulfate was changed to 0.028 g (0. 102 mmol) and the addition amount of ethylene glycol diglycidyl ether was changed to 0.005 g (0.026 mmol), 0.129 g (0.475 mmol) of 2,2'-azobis(2-amidinopropane) dihydrochloride was added as the azo-based compound in the second stage monomer aqueous solution, the addition amount of potassium persulfate was changed to 0.039 g (0.143 mmol), and the amount of water extracted to the outside of the system by azeotropic distillation was changed to 219.8 g.

Comparative Example 3

500 g or more of water absorbent resin particles C (median particle diameter 380 μm) were obtained in the same manner as in Comparative Example 1 except that 2.21 g (0.029 mol) of propylene glycol was used as the surface crosslinking agent.

Comparative Example 4

104.16 g of an emulsion (NIPPON A&L, SR-115) containing, in a water-dispersed state, a polymer including styrene and butadiene as constituent units with a non-volatile portion of 48% by mass was added to a polybeaker having an internal volume of 1 L. Thereafter, dilution was carried out by adding 395.84 g of ion-exchanged water to obtain a coating liquid.

500.0 g of the water absorbent resin particles A were injected into a container of a fluidized bed granulator and hot air at 50°C was blown from the bottom of the container. Subsequently, the coating liquid was sprayed for 90 minutes onto the water absorbent resin particles A being blown up by the air and the air supply was continued for 30 minutes after the spraying was finished. After drying, 503.3 g of coated resin particles having a median particle diameter of 352 μm were obtained.

5 g of the coated resin particles were weighed in a stainless-steel Petri dish having a diameter of 10 cm and a depth of 2 cm, coated with perforated aluminum foil, and heated under a condition of 80°C for 30 minutes using a hot air dryer (ADVANTEC, FV-320).

Example 1

501.5 g of coated resin particles having a median particle diameter of 355 μm were obtained in the same manner as in Comparative Example 4, except that 113.63 g of an emulsion (DSM Resins & Functional Materials, NeoCryl-A1127) containing, in a water-dispersed state, a polymer including acrylic acid as a constituent unit with a non-volatile portion of 44% by mass was diluted with 386.37 g of ion-exchanged water to obtain the coating liquid.

Example 2

500.2 g of coated resin particles having a median particle diameter of 348 μm were obtained in the same manner as in Comparative Example 4, except that 185.19 g of an emulsion (Mitsui Chemicals, Inc., Chemipearl S100) containing, in a water-dispersed state, a polymer including ethylene and acrylic acid as constituent units with a non-volatile portion of 27% by mass was diluted with 314.81 g of ion-exchanged water to produce a coating liquid.

Example 3

503.6 g of coated resin particles having a median particle diameter of 353 μm were obtained in the same manner as in Comparative Example 4, except that 111.11 g of an emulsion (DSM Resins & Functional Materials, NeoCryl XK-188) containing, in a water-dispersed state, a polymer including styrene and acrylic acid as constituent units with a non-volatile portion of 45% by mass was diluted with 388.89 g of ion-exchanged water to produce a coating liquid.

Example 4

537.9 g of coated resin particles having a median particle diameter of 358 μm were obtained in the same manner as in Comparative Example 4, except that 222.22 g of an emulsion (DSM Resins & Functional Materials, NeoCryl XK-188) containing, in a water-dispersed state, a polymer including styrene and acrylic acid as constituent units with a non-volatile portion of 45% by mass was diluted with 277.78 g of ion-exchanged water to produce a coating liquid.

Example 5

500 g or more of a water absorbent resin D was obtained by carrying out the same procedure as Comparative Example 2 except that the amount of water extracted to the outside of the system by azeotropic distillation was changed to 234.2 g.

Thereafter, 505.2 g of coated resin particles having a median particle diameter of 359 μm were obtained in the same manner as in Example 3, except that the resin particles were the water absorbent resin D.

<Elasticity Measurement>

0.200 g of a water absorbent resin was precisely weighed into an acrylic cylinder having an inner diameter of 2.0 cm and a depth of 5.0 cm, in which the bottom surface was lined with nylon mesh (Nippon Tokushu Fabric, NNo. 250T), the water absorbent resin was spread evenly on the bottom surface, and 13 g of physiological saline was poured in from the top of the acrylic cylinder. After 2 minutes, the acrylic cylinder was lifted up and excess physiological saline that was not absorbed was discharged from the bottom of the cylinder to obtain particles where liquid absorption occurred (sample for elasticity evaluation). An acrylic column having a diameter of 2 cm and a height of 6 cm was connected to the load cell of a small tabletop tester (SHIMADZU, EZ-TEST). The sample for elasticity evaluation was compressed from above at a speed of 1 cm/min using the load cell and the compression was continued until the stress from the sample for elasticity evaluation corresponded to 45N. Thereafter, the distance and the stress when pressing in the sample for elasticity evaluation were plotted and the elasticity (kPa/mm) was calculated from the slope of a straight line connecting the pressing in distance at a stress of 24 kPa (7.5 N) and the pressing in distance at a stress of 96 kPa (30 N).

<Measurement of Water retention capacity >

After adding 500 g of physiological saline at 25°C to a 500 mL polyethylene beaker, using a stirrer, 2.00 g of particles for evaluation were added little by little while rotating the stirrer at a rotation speed of 600 rpm. After the addition of the total amount of the particles for evaluation was finished, stirring was carried out for 30 minutes. Subsequently, the mixture was transferred into a cotton bag and the top of the cotton bag was closed using a rubber band. Subsequently, centrifugation (167G) was performed for 1 minute using a centrifuge. After dehydration, the mass WA was measured. The same operation was carried out without putting the particles for evaluation into the cotton bag and the mass WB of the empty cotton bag was measured. Then, the water retention capacity of physiological saline was calculated using the following formula.

Water retention capacity [g/g]=(WA-WB)/mass of particles for evaluation (=2.00)

<Production of Absorbent>

An absorbent core having a size of 40 cm x 12 cm was produced by using air papermaking to carry out even mixing using 12.0 g of the particles for evaluation and 8.0 g of crushed pulp (Rayonier, RAYFLOC). Subsequently, in a state where the top and bottom of the absorbent core were interposed between two sheets of tissue paper having the same size as the absorbent core and a basis weight of 16 g/m$^2$, pressing was carried out by applying a load of 141 kPa to the entire body for 30 seconds to produce an absorbent in which the content ratio of the particles for evaluation was 60% by mass. Further, an air-through type porous liquid permeable sheet made of polyethylene having the same size as the absorbent and a basis weight of 22 g/m$^2$ was arranged on the upper surface of the absorbent to obtain an absorbent article.

<Diffusion Distance Evaluation>

0.5 g of polyoxyethylene (10) octylphenyl ether (FUJIFILM Wako Pure Chemical Corporation), 200 g of sodium chloride (NACALAI TESQUE, INC.), 6 g of calcium chloride dihydrate (Kanto Chemical Co. Inc.), 12 g of magnesium chloride hexahydrate (Kanto Chemical Co. Inc.), 0.5 g of Food Blue No. 1 (Daiwa Dyestuff Mfg. Co., Ltd.), and 19781 g of ion-exchanged water were mixed to obtain a test solution. The absorbent article was placed on a horizontal table, a liquid injection cylinder having an opening having an inner diameter of 3 cm was placed in the center of the absorbent article, 80 mL of the test solution was injected into the cylinder at one time, and, after confirming that the liquid was completely absorbed, the liquid injection cylinder was removed. Similar liquid injection operations were performed at 30 minute intervals, for a total of three liquid injection operations. After the third liquid injection was finished, the result was left to stand for 15 minutes and the air-through type porous liquid permeable sheet made of polyethylene arranged on the upper surface was removed. The length of the blue region wetted by the test solution in the longitudinal direction of the absorbent was measured and set as the diffusion distance (cm).

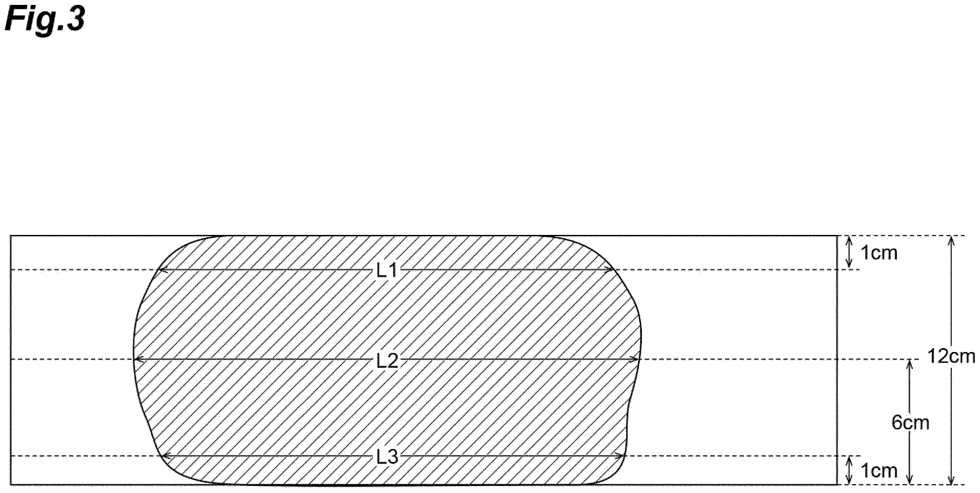

Specifically, as shown in Fig. 3, the diffusion distance (cm) is the average value of the length (L1) of the portion overlapping a hatched region (shown as a blue region in the absorbent wetted by the test solution) of a hypothetical line passing 1 cm inward from one end of the absorbent in the shortitudinal direction and parallel to the longitudinal direction of the absorbent, the length (L2) of the portion overlapping the hatched region of a hypothetical line passing through the center of the absorbent and parallel to the longitudinal direction of the absorbent, and the length (L3) of the portion overlapping the hatched region of a hypothetical line passing 1 cm inward from the other end of the absorbent in the shortitudinal direction and parallel to the longitudinal direction of the absorbent.

<Evaluation of Re-wet (RW)>

After performing the above-described diffusion distance evaluation (15 minutes after injecting the test solution), the removed air-through type porous liquid permeable sheet made of polyethylene was rearranged and the absorbent was left to stand for 45 minutes. Subsequently, a 10 cm square filter paper for which the mass (approximately 75 g) was measured preliminarily was placed near the test solution injection position on the absorbent article and a weight having a mass of 5.0 kg (approximately 0.7 psi) and a bottom surface of 10 cm x 10 cm was placed thereon. After loading for 5 minutes, the mass of the filter paper was measured and the increased mass was taken as the re-wet amount (g).

<Measurement of Median Particle Diameter>

Using a continuous fully automatic sonic vibration type sieving measuring device (Robot Shifter RPS-205, manufactured by Seishin Enterprise Co., Ltd.), sieves with JIS standard openings of 710 $\mu$m, 600 $\mu$m, 500 $\mu$m, 425 $\mu$m, 300 $\mu$m, 250 $\mu$m, and 150 $\mu$m, and a tray, the particle size distribution of 5 g of the particles for evaluation was measured. The relationship between the sieve openings and the integrated value of the mass percentage of particles remaining on the sieve was plotted on logarithmic probability paper by integrating the particle size distributions on the sieve in descending order of particle diameter. By connecting the plots on the probability paper with a straight line, the particle diameter corresponding to a cumulative mass percentage of 50% by mass was obtained as the median particle diameter.

<Measurement of Water Absorption Amount Under Load (AUL)>

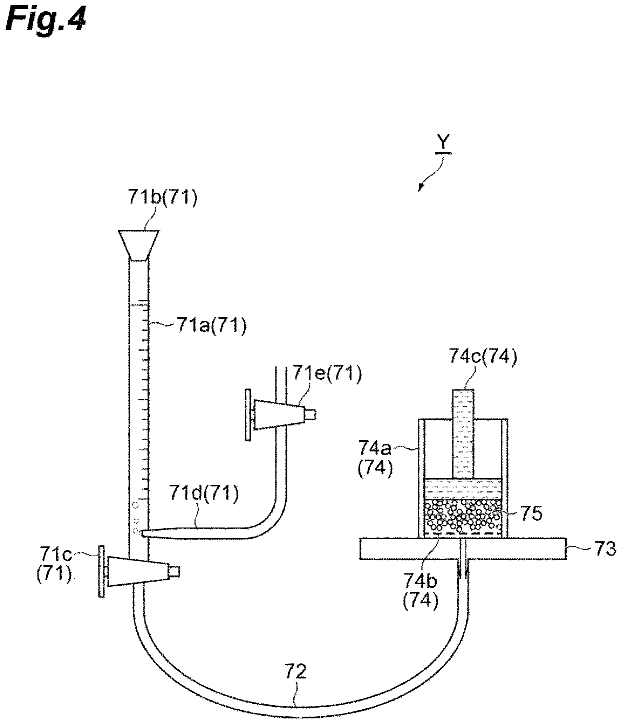

The amount of water absorbed by the water absorbent resin particles with respect to physiological saline under a load of 0.9 psi was measured using a measurement device Y shown in Fig. 4 in an environment of temperature 25°C$\pm$2°C and humidity 50$\pm$10%. The measurement device Y is formed of a burette unit 71, a conduit 72, a measurement table 73, and a measurement unit 74 placed on the measurement table 73. The burette unit 71 has a burette 71a extending in the vertical direction, a rubber stopper 71b arranged at the upper end of the burette 71a, a cock 71c arranged at the lower end of the burette 71a, an air introduction tube 71d for which one end extends into the burette 71a in the vicinity of the cock 71c, and a cock 71e arranged at the other end side of the air introduction tube 71d. The conduit 72 is attached between the burette unit 71 and the measurement table 73. The inner diameter of the conduit 72 is 6 mm. A hole having a diameter of 2 mm is formed in the central portion of the measurement table 73 and the conduit 72 is connected to the hole. The measurement unit 74 has a cylinder 74a (made of acrylic resin (plexiglass)), a nylon mesh 74b adhered to the bottom of the cylinder 74a, and a weight 74c. The inner diameter of the cylinder 74a is 20 mm. The openings of the nylon mesh 74b are 75 $\mu$m (200 mesh). At the time of measurement, water absorbent resin particles 75 to be measured are evenly scattered on the nylon mesh 74b. The diameter of the weight 74c is 19 mm and the mass of the weight 74c is 175.7 g. The weight 74c is placed on the water absorbent resin particles 75 and is able to apply a load of 0.9 psi to the water absorbent resin particles 75.

After putting 0.100 g of the water absorbent resin particles 75 into the cylinder 74a of the measurement device Y, the weight 74c was placed thereon and measurement was started. Since the same volume of air as the physiological saline absorbed by the water absorbent resin particles 75 is quickly and smoothly supplied to the inside of the burette 71a from the air introduction tube, the amount of reduction in the water level of the physiological saline inside the burette 71a corresponds to the amount of physiological saline absorbed by the water absorbent resin particles 75. The scale of the burette 71a is engraved from top to bottom in increments of 0 mL to 0.5 mL. As the water level of the physiological saline, the scale Va of the burette 71a before the start of water absorption and the scale Vb of the burette 71a 60 minutes after the start of water absorption were read and the water absorption amount under load (the water absorption amount with respect to physiological saline under a load of 0.9 psi) was calculated using the following formula.

$$\text{Water absorption amount under load [mL/g]} = (Vb\text{-}Va)/0.1$$

[Table 1]

| | Water absorbent resin particle performance | | | Absorbent performance | |
|---|---|---|---|---|---|
| | AUL 0.9 psi (mL/g) | Elasticity (kPa/mm) when pressed from 24 kPa (7.5 N) to 96 kPa (30 N) | Water retention capacity (g/g) | Diffusion area (cm) after 3 injections | RW (g) |
| | | 60 to 200 | 30 to 60 | 28 or more | 33 or less |
| Comparative Example 1 | 12.0 | 9 | 42 | 24 | 33 |

(continued)

| | Water absorbent resin particle performance | | | Absorbent performance | |
| --- | --- | --- | --- | --- | --- |
| | AUL 0.9 psi (mL/g) | Elasticity (kPa/mm) when pressed from 24 kPa (7.5 N) to 96 kPa (30 N) | Water retention capacity (g/g) | Diffusion area (cm) after 3 injections | RW (g) |
| | | 60 to 200 | 30 to 60 | 28 or more | 33 or less |
| Comparative Example 2 | 15.0 | 55 | 41 | 24 | 14 |
| Comparative Example 3 | 17.5 | 66 | 25 | 35 | 54 |
| Comparative Example 4 | 15.0 | 24 | 41 | 21 | 30 |
| Example 1 | 13.5 | 189 | 38 | 33 | 28 |
| Example 2 | 10.0 | 176 | 36 | 32 | 20 |
| Example 3 | 16.0 | 90 | 39 | 33 | 25 |
| Example 4 | 11.0 | 171 | 37 | 35 | 20 |
| Example 5 | 14.5 | 150 | 54 | 31 | 9 |

It was shown that the water absorbent resin particles of the Examples provide an absorbent that has better liquid diffusivity after liquid absorption and is able to suppress re-wet after liquid absorption.

**Reference Signs List**

10: Absorbent
10a, 75: Water absorbent resin particles
10b: Fiber layer
20a, 20b: Core wrap
30: Liquid permeable sheet
40: Liquid impermeable sheet
71: Burette unit
71a: Burette
71b: Rubber stopper
71c, 71e: Cock
71d: Air introduction tube
72: Conduit
73: Measurement table
74: Measurement unit
74a: Cylinder
74b: Nylon mesh
74c: Weight
100: Absorbent article
Y: Measurement device

**Claims**

1.  Water absorbent resin particles,
    having an elasticity of 60 to 200 [kPa/mm] when compressed at 24 kPa to 96 kPa after immersion in physiological saline for 2 minutes, and a water retention capacity of physiological saline of 30 to 60 [g/g].

2.  The water absorbent resin particles according to claim 1,

wherein the water absorbent resin particles comprise polymer particles, and
a coating layer that coats at least a part of the surface of the polymer particles.

3. The water absorbent resin particles according to claim 2,
   wherein the coating layer comprises a polymer having a carboxyl group.

4. An absorbent comprising:
   the water absorbent resin particles according to any one of claims 1 to 3.

*Fig.1*

EP 4 442 735 A1

*Fig.2*

100

30  20a

10b ⎱
10a ⎰ 10

20b

40

*Fig.3*

16

*Fig.4*

# INTERNATIONAL SEARCH REPORT

| | International application No. |
|---|---|
| | **PCT/JP2022/037928** |

**A. CLASSIFICATION OF SUBJECT MATTER**

*C08J 3/12*(2006.01)i; *A61F 13/53*(2006.01)i; *B01J 20/26*(2006.01)i; *B01J 20/28*(2006.01)i
FI:   C08J3/12 Z CEY; A61F13/53 300; B01J20/26 D; B01J20/28 Z

According to International Patent Classification (IPC) or to both national classification and IPC

**B. FIELDS SEARCHED**

Minimum documentation searched (classification system followed by classification symbols)
    C08J3/12; A61F13/53; B01J20/26; B01J20/28

Documentation searched other than minimum documentation to the extent that such documents are included in the fields searched

    Published examined utility model applications of Japan 1922-1996
    Published unexamined utility model applications of Japan 1971-2022
    Registered utility model specifications of Japan 1996-2022
    Published registered utility model applications of Japan 1994-2022

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| A | WO 2021/117780 A1 (SUMITOMO SEIKA CHEMICALS CO., LTD.) 17 June 2021 (2021-06-17)<br>    whole document | 1-4 |
| A | WO 2020/184389 A1 (SUMITOMO SEIKA CHEMICALS CO., LTD.) 17 September 2020 (2020-09-17)<br>    whole document | 1-4 |
| A | JP 2018-164732 A (SDP GLOBAL CO., LTD.) 25 October 2018 (2018-10-25)<br>    whole document | 1-4 |
| P, A | WO 2022/209972 A1 (SUMITOMO SEIKA CHEMICALS CO., LTD.) 06 October 2022 (2022-10-06)<br>    whole document | 1-4 |

☐ Further documents are listed in the continuation of Box C.    ☑ See patent family annex.

| | |
|---|---|
| * Special categories of cited documents:<br>"A"   document defining the general state of the art which is not considered to be of particular relevance<br>"E"   earlier application or patent but published on or after the international filing date<br>"L"   document which may throw doubts on priority claim(s) or which is cited to establish the publication date of another citation or other special reason (as specified)<br>"O"   document referring to an oral disclosure, use, exhibition or other means<br>"P"   document published prior to the international filing date but later than the priority date claimed | "T"   later document published after the international filing date or priority date and not in conflict with the application but cited to understand the principle or theory underlying the invention<br>"X"   document of particular relevance; the claimed invention cannot be considered novel or cannot be considered to involve an inventive step when the document is taken alone<br>"Y"   document of particular relevance; the claimed invention cannot be considered to involve an inventive step when the document is combined with one or more other such documents, such combination being obvious to a person skilled in the art<br>"&"   document member of the same patent family |

| Date of the actual completion of the international search | Date of mailing of the international search report |
|---|---|
| **07 December 2022** | **20 December 2022** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

**INTERNATIONAL SEARCH REPORT**
Information on patent family members

International application No.

**PCT/JP2022/037928**

| Patent document cited in search report | | | Publication date (day/month/year) | Patent family member(s) | | | Publication date (day/month/year) |
|---|---|---|---|---|---|---|---|
| WO | 2021/117780 | A1 | 17 June 2021 | WO | 2021/117781 | A1 | |
| | | | | WO | 2021/117782 | A1 | |
| | | | | WO | 2021/117783 | A1 | |
| | | | | WO | 2021/117784 | A1 | |
| | | | | WO | 2021/117785 | A1 | |
| | | | | WO | 2021/117786 | A1 | |
| WO | 2020/184389 | A1 | 17 September 2020 | US | 2022/0143575 | A1 | |
| | | | | whole document | | | |
| | | | | EP | 3936533 | A1 | |
| | | | | CN | 113544164 | A | |
| | | | | KR | 10-2021-0137068 | A | |
| JP | 2018-164732 | A | 25 October 2018 | WO | 2018/181277 | A1 | |
| | | | | CN | 110475610 | A | |
| WO | 2022/209972 | A1 | 06 October 2022 | (Family: none) | | | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2013095822 A
- JP H853550 A
- JP H8113653 A